# EUROPEAN PATENT SPECIFICATION

(11) **EP 3 890 677 B1**
(45) Date of publication and mention of the grant of the patent: **04.01.2023**
(21) Application number: 19818488.9
(22) Date of filing: 25.11.2019
(51) Int. Cl.: A61J 1/05, A61J 1/14

(54) **APPARATUS FOR DELIVERY OF RADIOEMBOLIZATION MICROSPHERES**
VORRICHTUNG ZUR ABGABE VON RADIOEMBOLISATIONSMIKROSPHÄREN
APPAREIL D'ADMINISTRATION DE MICROSPHÈRES DE RADIOEMBOLISATION

(30) Priority: 03.12.2018 US 201862774620 P; 10.10.2019 US 201962913461 P
(43) Date of publication of application: 13.10.2021
(73) Proprietor: Sirtex Medical Inc., Woburn, Massachusetts 01801 (US)
(72) Inventor: DRAKE, Jesse, Westborough, Massachusetts 01581 (US); MCCARTHY, Justin, Boxborough, Massachusetts 01719 (US); PARROTT, David, Cincinnati, Ohio 45223 (US); ROSE, Joshua, Natick, Massachusetts 01760 (US); THRAILKILL, Patrick T., Utica, New York 13501 (US); GALLAGHER, Charlotte A., milton, Massachusetts 01982 (US); CUTULI, Kate, Salem, New Hampshire 03079 (US); THOMPSON, Diana Sulas, Chicago, Illinois 60632 (US)
(74) Representative: Brand Murray Fuller LLP
(86) International application number: PCT/US2019/063000
(87) International publication number: WO 2020/117524

(56) References cited:
- US-A1- 2002 115 980
- US-A1- 2006 047 151
- US-A1- 2007 144 355
- US-A1- 2013 112 016

## Description

### CROSS-REFERENCE TO RELATED APPLICATIONS

This application claims any and all benefits as provided by law including benefit under 35 U.S.C. § 119(e) of the U.S. Provisional Application No. 62/774,620, filed December 3, 2018, and U.S. Provisional Application No. 62/913,461, filed October 10, 2019,

### FIELD OF THE INVENTION

The present disclosure relates to systems for embolization of a patient. More specifically, to vials for containing microspheres as well as systems for delivering the contained microspheres to a patient for radioembolization. Such a device is disclosed in the US2013112016.

### BACKGROUND

Colorectal cancer (CRC) is the fourth biggest cancer killer in the world, following lung, liver and stomach cancers respectively. In Europe and North America, it is the second most common cause of death from cancer. In Asia, it is the fourth most common cause of death from cancer (GLOBOCAN 2012; WWW.globocan.iarc.fr/Default.aspx, last accessed February 2017. The liver is the most common part of the body to which colorectal cancer spreads. This is because the colon is directly connected to the liver by blood vessels ( Clark ME et al. J Gastrointest Oncol 2014; 5: 374-387). Colorectal cancer can also spread to other organs such as the lungs or bones. Once the cancer starts to spread like this it is known as secondary or metastatic colorectal cancer, which is often abbreviated to mCRC.

Surgical removal (also known as resection) of the liver cancers currently provides the only realistic possibility of providing a cure for patients with tumors that have spread from the colon. However, approximately 20-30% of patients will have liver tumors that can be removed surgically. Of those patients that can be treated using resection, 15-67% (median 30%) have been reported to be alive after five years, compared to 0-6% for patients who do not undergo surgery. (Kopetz S et al. J Clin Oncol. 2009; 27(22): 3677-3683; Simmonds PC et al Br J Cancer. 2006 94(7): 982-99).

Although some patients with liver cancer can benefit from resection, many patients are not candidates given the degree of liver disease and advanced stage of their cancer at diagnosis. Although liver transplantation can be a cure, this is often not a viable option since globally, and even in the developed world, only a small fraction of all potential patients have access to transplantation. For these patients, local ablative therapies, including radiofrequency ablation (RFA), chemoembolization, and potentially novel chemotherapeutic agents, may extend life and provide palliation. (Natthida Khajornjiraphan et al. "Yttrium-90 Microspheres: A Review of Its Emerging Clinical Indications" Liver Cancer 2015; 1:6-15; Aaron K. T. Tong et al. "Yttrium-90 hepatic radioembolization: clinical review and current techniques in interventional radiology and personalized dosimetry", Br J Radiol; 2015; 89: 2-16.)

Radioembolization, also known as Selective Internal Radiation Therapy (SIRT) is a relatively new procedure with good overall survival and is a recommended treatment for some patients with intermediate to late stage liver cancer. SIRT involves embolizing an artery feeding a target tumor with a radioactive particle so that the tumor is selectively irradiated. SIRT uses particles or microspheres containing yttrium 90 (Y90), which is a pure beta emitter Y90 having a 2.6-day half-life and a tissue penetration depth of about 2.5 mm in the liver. SIRT is performed using a minimally invasive surgical technique by an interventional radiologist using commercially available devices.

Although effective, the commercial devices for SIRT can be cumbersome to use. For example, some procedures and devices include steps where a stopcock is manually positioned to alternate between a position for delivering radioactive particles to a position for delivering water or imaging solution. A drawback to this is that in practice the radiologist must remember to change the stopcock position or the incorrect fluid will be delivered. In addition, the particles tend to be denser than the delivery solution (e.g., DI water) and tend to quickly settle at the bottom of the delivery vial. To ensure delivery of the particles the particles are disturbed by energetic bursts of incoming delivery solution so that the particles are lofted up and can be ejected from the delivery vial through an egress tube. Although useful, this method relies on the manual positioning of the tube delivering solution to the vile (e.g. using a needle), in the delivery vials by trial and error to ensure the particles are lofted up into the solution. In addition, the devices include several tubes, syringes, a catheter, connectors as well as the delivery vial to manage during preparation of the procedure, during the procedure and after the procedure. Managing these components is very important, especially since the radioactive contamination of some of the components is inherent in the procedure. Although effective, these also deliver microspheres in non-uniform pulses from the vial to the catheter.

There is therefore a need for easier to use devices for the delivery of radioactive particles in the practical application of SIRT. This disclosure addresses some of these needs.

### SUMMARY

In general, the disclosure herein relates to systems for delivery of microspheres as further disclosed in claim 1. (e.g., radioembolization microspheres). The systems can include vials (for containing the microspheres) having an inclined base or a domed base, as well as inlets having controlled angles of entry which aid in dispersing the microspheres in a carrier fluid to facilitate delivery to a patient. The system can include a tubing configuration including connectors and one-way valves in order provide a microsphere delivery system that is easy to operate, such as by an interventional radiologist.

In one aspect the disclosure includes a vial having a bottom interior surface at least a portion of which has an incline angle to a horizontal plane greater than 0 degrees and a cylindrical interior wall substantially perpendicular to the horizontal plane and meeting the bottom interior surface, wherein the bottom surface and cylindrical wall form a container for containing a liquid; (e.g., the walls can have 0-10 degrees of draft, 2-5 degrees of draft due to fabrication processes, such as molding). The vial can also have an inlet channel for fluid flow in to the vial through the cylindrical wall and an outlet channel for fluid flow out of the vial through the cylindrical wall, the inlet channel defining a direction vector of fluid flow, wherein the direction vector is oriented at a first predefined angle with respect to the horizontal plane and oriented at a second predefined angle with respect to a line tangent to the cylindrical interior wall such that fluid flowing in through the inlet channel causes a rotational flow or an upwardly moving fluid vortex to be created in the vial.

Optionally, the inlet channel of the vial is disposed at a vertical position closer to the bottom interior surface than the outlet channel. Optionally, the cylindrical wall includes a ramp portion that extends inwardly from the cylindrical wall and further defines an interior surface that pushes rotational flow in the vial toward a center of the vial and wherein the ramp portion extends vertically from a position proximate to the bottom interior surface to a first predefined vertical position with respect to the outlet channel. Optionally, the ramp portion is configured to produce a higher mixing shear in a solution in a first portion of the vial in a region proximate to the inlet channel and outlet channel as compared to lower mixing shear in a solution in a second portion of the vial in regions above outlet channel or above the ramp portion. Optionally, the inlet channel comprises an inner diameter equal to or smaller than an inner diameter of the outlet channel (e.g., 1% to 80%, from about 10% to about 75%, from about 25% to about 50% smaller). Optionally, the outlet port has an inner diameter between about 1/32" and ¼" inches.

In some other embodiments of the vial, the bottom interior surface is approximately flat and the first predefined angle is between about 10 and 60 degrees (e.g., in a range from 20-25 degrees) with respect to the horizontal plane. Optionally, the second predefined angle of the direction vector of fluid flow through the inlet is equal to or greater than 0° (e.g., between 0 and 20°, between 0 and 10°) with respect to the line tangent to cylindrical wall.

Optionally the vial has a convex interior bottom surface. In some other options, a radius of curvature of the cylindrical wall is greater than a radius of curvature of the convex bottom surface.

In another aspect, the disclosure comprises a delivery device comprising a first syringe in fluid connection to the inlet of the vial as described herein, wherein the vial is in fluid connection to a three-way fluid connector through the outlet channel, a second syringe in fluid connection to the three-way fluid connector, and a patient interface in fluid connection to the three-way fluid connector. Optionally, the device is configured for delivery of (a) a first solution from the first syringe, through the vial, through the three-way fluid connector, and through the patient interface to a patient, and (b) a second solution from the second syringe, through the three-way connector, and through the patient interface to a patient; wherein the inlet channel is disposed for delivering the first solution to the vial at the first predefined angle such that settled particles in a particle-containing solution in the vial are dispersed in the solution and exit the vial through the outlet channel. Optionally, the patient interface is a microcatheter, for example, a microcatheter for radioembolization.

Optionally, the device further comprises a selecting-valve integrated with the three-way fluid connector for selective delivery of the first solution from the first syringe through the selecting valve to the patient or for selective delivery of the second solution from the second syringe through the selecting valve to the patient. Optionally, the selecting-valve integrated with the three-way fluid connector is configured as a three-way stopcock.

Optionally, the delivery device comprises a first one-way valve disposed between the first syringe and the inlet channel, and a second one-way valve disposed between the second syringe the three-way connector, wherein the first one-way valve allows fluid flow from the first syringe to the vial, and the second one-way valve allows fluid flow from the second syringe to the three-way fluid-connector. Optionally, the device further comprises a third one-way valve disposed between the vial outlet and the three-way fluid connector, wherein the third one-way valve allows fluid and particle flow from the vial to the patient interface when the fluid pressure on an inlet of the third one-way valve above the pressure on an outlet of the third one-way valve, for example, for some valves in the range from about 0.1 to about 1.0 psi, such as about 0.2 - 0.4 ps (1 psi = 6894,76 Pascal).

Optionally, the delivery device further includes a vial holder configured as a container having a top opening and a removable top cover, and wherein the container is configured to receive the vial therein through the top opening, and the removable top cover is configured for compressing an elastomeric seal against an opening at the top of the vial when the top cover is attached to the container, thereby providing a fluid seal at the top opening of the vial. Optionally, the inlet channel is provided with fluid connection to the first syringe using a first tube, the outlet channel is provided with fluid connection to the three-way valve using a second tube, and the vial holder includes an opening for allowing connection of the first tube to the inlet channel, and an opening for allowing connection of the second tube to the outlet channel. Optionally, the device further comprises a light disposed to illuminate the interior of the vial and wherein the vial and vial container are at least partially (e.g., at least 80%, e.g., at least 99%, at least about 100%). Optionally, the container and vial are at least partially opaque to ionizing radiation (e.g., at least 50% of the radiation emanating from the interior of the vial is attenuated by the vial and container). For example, the vial includes a shielding that is between about 1/8" and ½" thick acrylic (e.g., about ¼" acrylic) (1 inch=25,4 mm).

Optionally, the delivery device further comprises a holder for the vial and vial holder. Optionally, the holder for the vial and vial holder includes a moat channel surrounding the vial and configured for containing at least the volume of the interior of the vial, a transparent lid for enclosing the vial and three-way fluid connector, and a removable tray configured for attachment to the holder and configured for holding the three-way fluid connector and removably attaching to the vial holder. Optionally, the fluid connection from the second syringe to the three-way fluid connector is provide by removable attachment to a third tube, the fluid connection from the three-way fluid connector to the patient interface is provide by removable attachment to a fourth tube, wherein at least a portion of the first, third and fourth tubes are placed in the holder when the device is in operation.

In yet another aspect, the disclosure comprises a method for delivery of radiomicrospheres to a patient in need of radioembolization. Optionally, the method uses the device as described herein to deliver radiomicrospheres from the vial as described herein to the patient. Optionally, the method further includes delivering a contrast agent from the second syringe to the patient and visualizing the artery using the delivered contrast agent. Optionally, the radiomicrospheres include Y⁹⁰.

The vial and devices described herein provide many improvements including ease of operation as well as improved delivery of microspheres during the delivery of radioactive particles, for example, in a radioembolization procedure.

### BRIEF DESCRIPTION OF THE FIGURES

This patent or application file contains at least one drawing executed in color. Copies of this patent or patent application publication with color drawings will be provided by the Office upon request and payment of the necessary fee.
**FIG. 1** is a diagrammatic view of a system for delivering microspheres such as radiomicrospheres, according to some embodiments.
**FIG. 2** is a diagrammatic view of a system for delivering microspheres, according to some alternative embodiments.
**FIG. 3A** is a front cross section view of an embodiment of a vial having an inclined base.
**FIG. 3B** is a diagrammatic view of the bottom of the vial having an inclined base.
**FIG. 3C** is a front cross section view of an embodiment of a vial having a domed base.
**FIG. 3D** is a diagrammatic view of the bottom of the vial having a domed base.
**FIG. 4A** is a top cross section view of an embodiment of a vial showing a tangential entry port.
**FIG. 4B** shows a diagrammatic view of the cylindrical wall of the vial in relation to a tangent line to the cylindrical wall and a direction vector of fluid flow.
**FIG. 5** is a front cross section view of an embodiment of a vial having an inclined base and depicting a rotational flow.
**FIG. 6A** is a cross section top view of a vial with a tangential entry port and featuring a ramp.
**FIG. 6B** is a cross section front view of a vial having an inclined base and a ramp.
**FIG. 7** shows a cross section front view of a vial with an inclined base illustrating the positioning of inlet and outlets with respect to particle/sphere level therein.
**FIG. 8** shows a cross section front view of a vial having an inclined base and illustrates the angle of the integrated inlet with respect to a horizontal plane.
**FIG. 9A** is a front cross section view showing a vial with tube guides and integrated inlet and outlet.
**FIG. 9B** is a front cross section view showing a vial with tubes placed in tube guides and integrated inlet and outlets.
**FIG. 9C** is an isometric view showing a vial with tubes placed in tube guides and integrated inlet and outlets.
**FIG. 10A** is a cross section view front view showing an embodiment of a vial in a holder with a top cover and sealing element.
**FIG. 10B** is a cross section front view showing another embodiment of a vial in a holder.
**FIG. 10C** is a blown up view showing an embodiment of a vial in a holder with a top cover and a sealing element.
**FIG. 10D** shows a secondary holder for a vial holder.
**FIG. 11** is an isometric view of an embodiment including a holder for a vial.
**FIG. 12A** is side front view of an embodiment of a holder including a tray for engagement with vial holder.
**FIG. 12B** is an isometric detailed view of an embodiment of a holder including a tray for engagement with vial holder.
**FIG. 12C** shows an isometric view of an embodiment of a tray and a T-connector.
**FIG. 13** shows an isometric of an embodiment of an apparatus for delivering radiomicrospheres.
**FIG. 14A** is an isometric view of another embodiment of an apparatus for delivering radiomicrospheres including a holder for vial.
**FIG. 14B** shows embodiment of tubes that can be used for delivery of microspheres.
**FIG. 15** is a diagrammatic view of the holder previously shown illustrating tube and vial holder placement.
**FIG. 16** is a diagrammatic view of the holder previously shown illustrating syringe and light source placement.
**FIG. 17** shows an isometric view of an embodiment of an apparatus for delivering radiomicrospheres.
**FIG. 18** shows an isometric view of another embodiment of an apparatus for delivering radiomicrospheres.
**FIG. 19** shows a blown up view of an apparatus for delivering radiomicrospheres.
**FIG. 20A** show a left side isometric view of an apparatus for delivering radiomicrospheres.
**FIG. 20B** show a right side isometric view of an apparatus for delivering radiomicrospheres.

### DETAILED DESCRIPTION

The present disclosure is directed to components, systems and methods for delivering microspheres or other elements that result in embolization of a blood vessel or other biological passageway. The system can include one or more vials that serve as a reservoir for delivering the microspheres to the patient through a configuration of tubing. In addition, disclosed herein are systems for managing and operating the vials in a safe and effective manner. In accordance with some illustrative embodiments of the, the vials and systems can be used to facilitate the administration of radiomicrospheres in a radioembolization procedure administered by an interventional radiologist to a patient, such as a patient having liver cancer.

Radioembolization or SIRT is an embolization procedure and requires administration of radiomicrospheres to a major artery feeding a tumor. Since radioisotopes have a half-life, the procedure must be scheduled and the amount of spheres delivered is dependent on the activity of the radioisotope at the time of delivery. For example, Y90 undergoes β- decay having 2.28 MeV decay energy and with a half-life of 2.6 days. Therefore, for Y90 radiomicrospheres the calibrated dose must provide the appropriate amount of GBq per treatment, with the number of microspheres varying depending on the prescribed dosage and how many days after creation of the Y90. Careful dosing is critical since under-dosing can be ineffective and require additional treatments, while over dosing can lead to unwanted radiation (e.g., to other organs) and toxicity to the patient. It is therefore important to carefully dose and administer radiomicrospheres during radioembolization.

Microspheres, such as radiomicrospheres, can include ceramic materials such as glass and polymeric beads, and, in the case of radiomicrospheres, encapsulate or are functionalized with, the radioisotope. In some embodiments, microspheres used are spherical in shape and have a diameter between about 5 and 100 µm (e.g., 20 -80 µm, 30-50 µm or about 35 µm). These microspheres can be delivered by an embolization microcatheter using a carrier fluid. The carrier fluid can include water and/or saline solutions. As used herein, carrier and delivery fluid or solution are equivalent terms. The microspheres are denser than the carrier solution and therefore, when the solution is quiescent, tend to settle at the bottom of a container holding the solution and microspheres. Delivery therefore requires the microspheres to be dispersed or re-dispersed into the carrier solution, so that they can be delivered through the catheter.

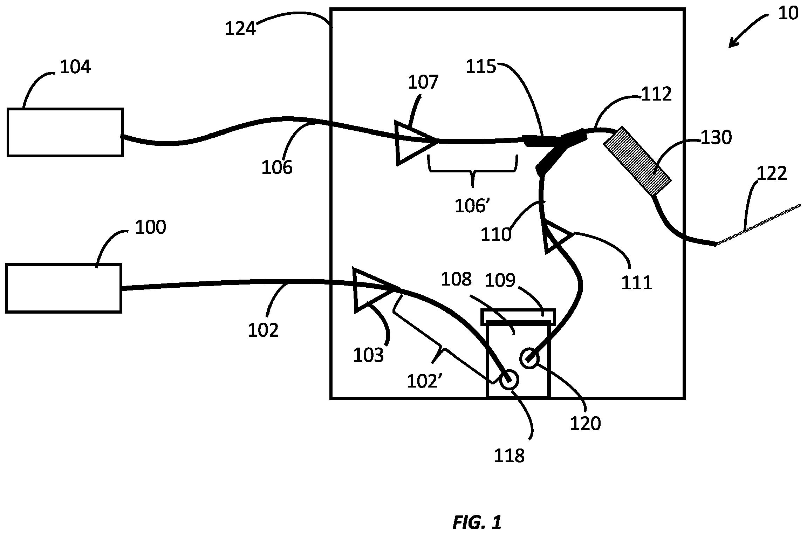

One embodiment of a system 10 for delivering microspheres (e.g., radiomicrospheres) is shown in FIG. 1. The system 10 can include a syringe 100 for delivery of DI water connected by line 102 to the vial or reservoir 108 containing the microspheres and the DI water delivered to the vial 108 can cause the microspheres to disburse and be delivered through line 110, and microcatheter 122 to the patient. In accordance with some embodiments, two syringes can be used. One syringe 100 containing DI water and another syringe 104 containing a non-ionic contrast agent can be connected to the system 10. The first syringe 100 with DI water can be connected by line 102 to the vial 108 containing the microspheres and the vial 108 can be connected by line 110 to a three-way connector 115 which is connected to the patient through the microcatheter line 112 and microcatheter 122 to the patient. The connector 115 can be any connector that has at least three openings such as a T connector or a Y connector. In some embodiments, the connector is a T connector. In some embodiments, the connector is a Y connector. The second syringe 104 containing non-ionic contrast agent can be connected by line 106 to the connector 115 which is connected to the patient through the microcatheter line 112 and microcatheter 122 to the patient. A first one-way valve 103 can be connected between the first syringe 100 and the vial 108 and a second one-way valve 107 can be connected between the second syringe 104 and the connector 115 to control the flow fluid in the system 10. A third one-way valve 111 can be connected between the vial 108 and the connector 115 to prevent backflow into the vial 108. In some embodiments, the vial 108 also includes a cap 109. The cap can be removable (e.g., a screw on cap) or it can be sealed at the top of the vial (e.g., crimped on). In some embodiments, the cap is crimped onto the vial. In some embodiments the vial (e.g., the vial cap 109) has a septum, such as a septum that can be pieced with a needle, such as for charging the vial with microspheres. In some embodiments the system 10 also includes a bubble trap 130. Bubble trap 130 ensures that no bubbles that pass through three-way connector 115 and into tube 112 pass to the microcatheter 122.

The one way valves can be of any kind and can be independently selected. For example, the valves can be one-way, check valves or Duckbill Valves (e.g., part 80065 available from Qosina Corp). The valves can have different or the same crack or opening pressures. For example, in some embodiments the valve 111 can have a crack pressure that is at least about 0.2 psi, such as between about 0.2 and 0.8 psi, or about 0.4 psi. Without being bound to a specific theory, a low crack pressure for valve 111 can reduce the shear stresses imparted on radiomicrospheres and minimize any fragmentation.

The system 10 can include an enclosure depicted as a box 124. The enclosure 124 can serve to hold some of the various components of system 10 therein and provide a shield for the operator during use of the system 10. Without limitation, the enclosure 124 can include transparent portions so as to allow viewing of components, as well including radiation opaque portions that can serve as shielding to the operator. In some embodiments, the holder is made using materials including radiation opaque material such as acrylic, wherein the thickness is sufficient for at least about 50% attenuation of ionizing radiation emanating from anywhere within the enclosure, such as from vial 108, and lines 102, 106, 112, and 110. Other materials can also be coated on the enclosure or used in construction of the holder. For example, high Z materials such as lead can be included. In some embodiments, the holder is at least about 50% transparent to visible light.

Although the one-way valve 103 can be placed anywhere along line 102, in some embodiments it is positioned so that the valve 103 is within enclosure 124. Likewise, although the one-way valve 107 can be placed anywhere along line 106, in some embodiments it is positioned so that the valve 107 is within enclosure 124. In an alternative embodiment, the three-way connector 115 can include one, two or three one-way valves that only permit flow into the ports connected to line 106 and line 110 and only permit flow out of the third port connected to line 112. In embodiments where the one-way valve 103 and 107 are configured to be within the enclosure 124, accidental exposure due to radioactive material flowing back towards syringe 100 or 104 can be minimized since the one-way valves prevent flow past regions 102' in the direction of syringe 100, and 106' in the direction of syringe 104.

In operation, a user (e.g., an interventional radiologist) inserts the transfemoral catheter 122 into the hepatic artery of the patient under x-ray guidance. The selective catheterization of the proper hepatic or lobar branches can be done using a large lumen microcatheter. It is useful that the radiomicrospheres are delivered selectively to the liver tumor and not delivered to other organs such as the pancreas, stomach or duodenum. Preferably, the microcatheter inserted into the hepatic artery is positioned distal to the gastroduodenal artery (GDA) to avoid the microspheres travelling to the duodenum or stomach. Other precautions can be taken to avoid the radiomicrospheres traveling to unintended locations. During the procedure, the radiologist can check periodically, e.g., using an imaging or contrast agent and x-ray, to confirm that the catheter is correctly placed and that no reflux is occurring.

Once the microcatheter 122 is correctly placed in the patient, the microsphere delivery enclosure 124 can be placed beside, and slightly higher than, the exam table with the patient. The patient can be positioned horizontally, for example in a supine position for the procedure. Syringe 104 containing DI water, and when used, contrast agent, can be connected to line 106 which is connected to three-way connection 115. Syringe 100 of DI water can be connected to the line 102 which is connected to the vial 108, and the vial 108 can be connected to the three-way connection 115 by line 110. The microcatheter line 112 can be used to connect the three-way connector 115 to the microcatheter 122 in the patient. The connections can, for example, include Luer type taper connections, although other fluid connections can be used.

Once the tubing lines are connected, the delivery of fluid and microspheres can begin. Initially, using syringe 104, 2-3 mL of DI water with contrast agent can be injected into the system 10 to check that line 112 and microcatheter 122 are securely connected and positioned. In accordance with some embodiments of the invention, using the first syringe 100, DI water can be delivered to the inlet channel 118 of the vial 108 (containing a predefined quantity of microspheres) in 0.25 to 0.5 ml pulses. The pulsation causes agitation of the microspheres in the vial 108 so that they are lofted up from the bottom of the vial 108 to the level of the outlet channel 120 and dispersed in solution so they can be delivered out through the outlet channel 120 and line 110 to the three-way connection 115 to the microcatheter line 112 which is connected to the microcatheter 122. In some embodiments, the injection is done slowly at a rate of not more than 5 mL/min. In some embodiments, pulsation is not needed and delivery is by a steady and uniform delivery of solution from the first syringe 100. In accordance with some embodiments, prior to the microspheres reaching the catheter, the second syringe 104 can be used to deliver 3-5 mL of DI water (e.g., in 0.25 to 0.5 ml pulses) to flush the microspheres through the catheter at a rate of not more than 5 mL/min. The slow and pulsed administration helps ensure even distribution and delivery of the radio microspheres to the hepatic artery and tumor and avoids reflux back up the hepatic artery. Antegrade flow to the tumor can be evaluated at any point during the procedure by administering DI water with a contrast agent through line 106 and using fluoroscopy.

The radioembolization procedure can be continued by alternatively using syringes 100 and 104 to deliver the microspheres to patient in multiple, small aliquots over about 20 minutes (or more or less time, depending on the prescribed treatment). The radiologist can periodically check the position of the microcatheter to ensure it remains correctly sited during the delivery procedure and confirms that blood is flowing forward without stasis. As the procedure progresses, the concentration of microspheres in the vial 108 decreases and the aliquot volumes of microspheres can be increased if there is good antegrade flow. After the procedure is completed, the microcatheter 122 can be removed and the patient closed.

When the reservoir is almost empty, the last of the microspheres can be ejected from the reservoir using air to clean out the lines. The final microspheres are then delivered by DI water delivered from the 102 line. The syringes can be disconnected from lines 102 and 106, and the lines can be capped. The 102 line, 106 line, 112 line, reservoir 108 as well as the three-way connector 115, and catheter 122 can be then treated as radioactive waste and disposed appropriately.

A scan of the upper abdomen, for example using a single-photon emission computerized tomography (SPECT) system, can be performed within 24 hours after implantation of the microspheres. The scan can help to confirm placement of the microspheres in the liver, for example, the SPECT scan will detect the Bremsstrahlung radiation from the Y90.

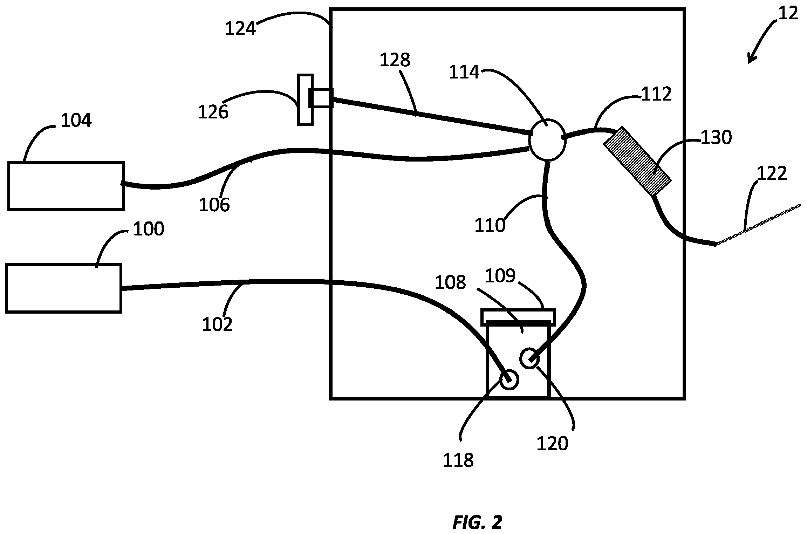

An alternative embodiment of a system 12 for delivering microspheres (e.g., radiomicrospheres) is shown in FIG. 2. System 12 is similar to system 10 shown in FIG. 1, except that the three-way connection 115 is replaced with a stopcock valve 114 that controls the source of fluid flow to the microcatheter line 112 and the microcatheter 122. The use of the stopcock obviates the need for the one-way valves 103, 107, 111 used in system 10. similarly to system 10, system 12 can include a first syringe 100 containing DI water and a second syringe 104 containing contrast agent and/or DI water. The first syringe 100 can be connected by line 102 to the inlet channel 118 of the vial 108. Line 110 can connect the outlet channel 120 of the vial 108 to the stopcock valve 114 and microcatheter line 112 can connect the stopcock valve 114 to the microcatheter 122. The vial 108 with connected lines 102, 110 and stopcock 114 can be encased in a microsphere delivery box 124 (e.g., a housing, holder or enclosure). The stopcock valve 114 can be operated by a control lever or knob 126 that can be connected to the stopcock valve 114 by extension rod 128 which positions the control knob 126 on the outside of the delivery box 124.

The operation of system 12 is similar to that of system 10 except that prior to injecting DI water from syringe 100 into the system 12, the stopcock valve 114 is placed in a first position that connects line 110 to the microcatheter line 112 and prior to injecting DI water and/or contrast agent from syringe 104 into the system 12, the stopcock valve 114 is placed in a second position that connects line 106 to the microcatheter line 112.

Some embodiments can include a combination of the features as depicted and described with reference to FIGS. 1 and 2. For example, some embodiments include a one-way valve 103, a one-way valve 107, a vial 108 having an inlet channel 118 and outlet channel 120, a stopcock valve 114 and any combination thereof.

It is understood the a "line" as used herein includes any useful element for transferring fluid including, for example, a tube such as a flexible plastic tube, but also can including couplers, rigid tubes, or channels. The lines can also be of any dimension although lines meant for delivery of microspheres must be larger than the microspheres and in some embodiments are at least about 2, 5, 10 or more times larger than the microspheres. In some embodiments, the lines have an inside diameter in a range from about 1/32 in. to 1/4 in.

In some embodiments, the syringes as described herein can be replaced by other fluid delivery devices such as a pump including a syringe pump, a displacement pump or a peristaltic pump.

In accordance with some embodiments, the vial 108 can include a cylindrical wall 414 and an inclined or domed base and the input channel 118 can be configured at a predefined angle to produce a tangential entry of fluid resulting in the formation of a vortex in the vial that causes microspheres sitting on the base to become suspended in solution above the base. In accordance with some embodiments, the cylindrical wall 414 can be substantially perpendicular to the base or bottom of the vial 108 (e.g., in a range from 92 degrees to 95 degrees in order to allow fabrication by injection molding). In accordance with some embodiments, the integrated input and output channels as well as the interior surfaces of the vial can be arranged and configured to create a rising vortex of disperse particles (e.g., microspheres) to a predefined level in the vial such that they can be delivered to the patient from the vial through the outlet channel.

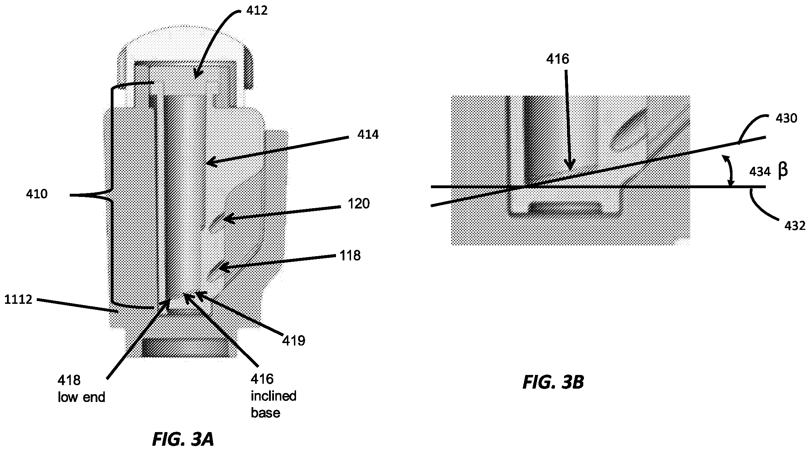

FIG. 3A is a front cross section view of one embodiment of a vial 108 having an inclined base 416. The vial 108 is shown in region 410 of holder 1112. An interior volume of the vial 108 can be defined by open top 412, a cylindrical wall 414 and an inclined bottom interior surface (e.g., inclined base) 416. In some embodiments, particles (e.g., microspheres and radiomicrospheres) that are denser than the liquid carrier (e.g., DI water, saline solution) settle in the low end 418 of the interior volume of the vial 108. The inlet channel 118 directs the fluid into the vial 108 at a position adjacent to the high end 419 of the inclined base 416. The entry trajectory or directional vector of fluid flow is determined, at least in part, by the predefined downward angle (with respect to the horizontal) and the predefined tangential angle (with respect to the cylindrical wall) of the input channel 118. The directional vector of fluid flow is directed toward the fluid and particles resting on and around the inclined base such that a rising vortex of the fluid and the particles is created. By selecting and configuring the predefined flow rates and directional vector angles, the particles can become suspended at a predefined height within the vial (e.g., the height of the outlet channel 120). The dispersed particles can flow out of the vial 108 through outlet channel 120 when the liquid carrier is injected (e.g., pulsed) into the vial 108 through inlet channel 118.

FIG. 3B is a detailed view of the bottom of the vial shown in FIG. 3A. The plane 430 which encompasses the plane defined by the interior surface 416 is shown relative to the horizontal plane 432. The planes 430 and 432 intersect and define an angle beta (β) 434. In accordance with some embodiments, the angle β can be in the range from about 10° to 60°. In some embodiments, the angle β is in the range from 20° to 25°. In accordance with some embodiments, the flow rate can be varied to control the rotational speed and height of the vortex which is dampened by the viscosity of the fluid. The flow rate can be controlled by the diameter of the inlet channel 118. In accordance with some embodiments, the microsphere size is fixed at production to 35 microns in diameter. In some embodiments the microspheres are between about 20 and about 80 microns, such as between about 25 and about 45 microns or between about 30 and 40 microns.

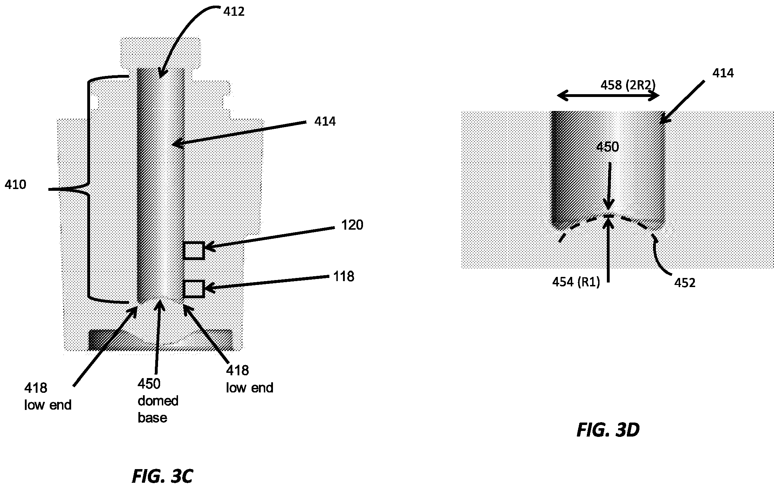

FIG. 3C is a front cross section view of an embodiment of a vial having a domed base 450. In this embodiment, the particles or radiomicrospheres settle and are collected along the perimeter of the vial base 450, which is the low end 418 of the interior volume of the vial 108. The entry trajectory or directional vector of the fluid flow is determined, at least in part, by the angle of the inlet channel 118. The tangential angle (with respect to the cylindrical wall 414) and horizontal angle (with respect to the horizontal plane) of the input channel 118 creates a rising vortex of the particles. The domed based 450 facilitates the rotational flow of the vortex which promotes mixing and homogeneity of the microspheres in the fluid volume contained in the vial 108.

FIG. 3D shows a detailed view of the bottom of the vial shown in FIG. 3C. The arc shown as a line 452 follows the projection of convex domed base 450 and has a radius of curvature R1 indicated by arrow 454. The cylindrical wall of the vial 414 has an associated radius of curvature R2, which is half of the diameter indicted by the two headed arrow 458. In some embodiments, the ratio of radius of curvature of the cylindrical wall R2 and radius of curvature of the convex bottom surface R1 is greater than or equal to 1:1. In accordance with some embodiments, the ratio can be selected such that a space or channel is formed along the cylindrical wall 414 such that the microspheres are able to collect in a predefined position with respect to the entry trajectory or directional vector of fluid flow from the inlet channel 118. While the domed base 450 is shown as having a circular curvature, in other embodiments the dome base 450 can have an elliptical curvature or any non-circular shape.

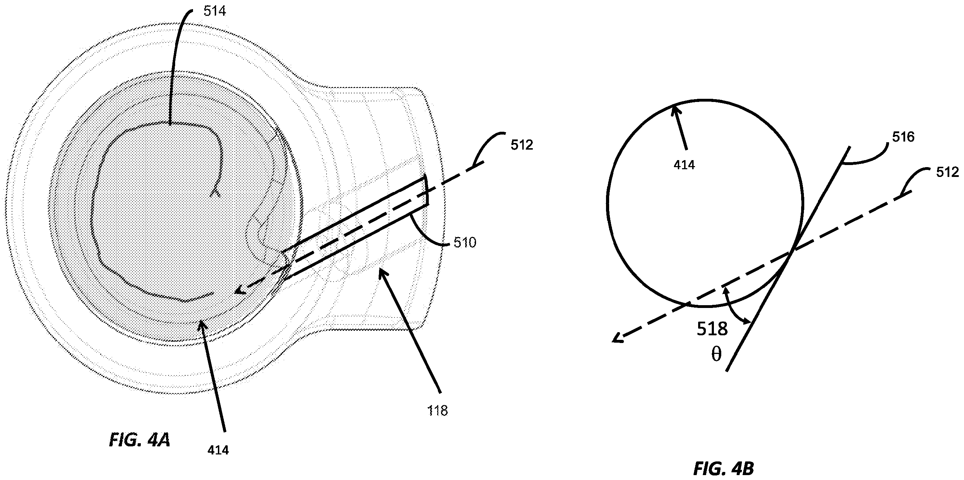

FIG. 4A is a top section view of an embodiment of a vial 108 showing a tangential inlet channel 118. The inlet channel 118 has a channel 510 that defines a direction vector of fluid flow 512 indicated by the dashed arrow. The tangential entry angle of the fluid from the inlet channel 118 creates a rotational flow as indicated by curved arrow 514.

As used herein, "tangential" includes vectors with angles up to about 20° from a geometric tangent. This definition is further illustrated with reference to FIG. 4B, showing a projection of the cylindrical wall 414 of a vial 108 onto a horizontal plane, and where the vial 108 is in the same orientation as depicted by FIG. 4A. FIG. 4B shows the tangent line 516 to the cylindrical wall 414 and the angle theta (θ) 518 of the direction vector of fluid flow 512 with respect to the tangent line 516. In accordance with some embodiments, the inlet channel 118 of the vial 108 provides a direction vector of fluid flow having an angle θ less than about 20°. In some embodiments, the angle θ can be in the range from about 20° to about 0° or in the range from about 10° to about 0°.

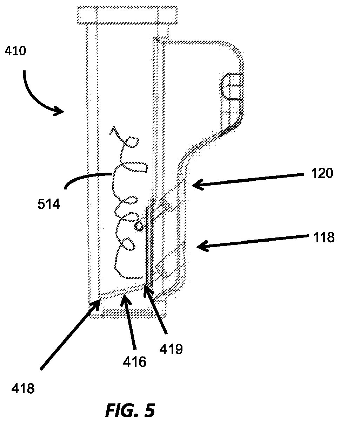

FIG. 5 is a front cross section view of an embodiment of a vial 108 having an inclined bottom interior surface 416 and depicting a rising rotational vortex flow 514 created by the fluid flow from the inlet channel 118. Without limitation to a particular mechanism, it is proposed that the rotating flow moves up the inclined surface 416, after reaching bottom 418, and creating the swirling flow or vortex 514 that includes an upward component that disburses the particles that have settled on the inclined surface 416. The upward component causes the disbursed particles to rise above the inclined surface 416 and the inlet channel 118 to the level of the outlet channel 120 to facilitate the flow of the particles out of the outlet channel 120 for delivery of the maximum quantity of particles to the patient. This can allow the delivery of a uniform distribution of microspheres into the line 112, through catheter 122 and to the patient, as compared to other methods that may utilize a pulsed delivery to loft the microspheres, leading to a less uniform distribution of the microspheres in line 112.

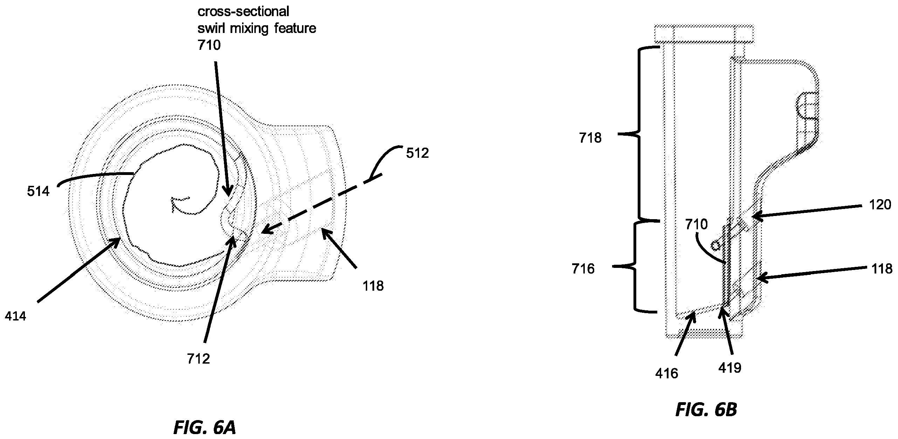

FIG. 6A shows a section top view of a vial 108 with a tangential inlet channel and including a ramp portion 710 according to some embodiments. The fluid enters the vial along direction vector of fluid flow 512 at the leeward side 712 of the ramp, after which the cylindrical inner wall 414 forces the flow in a circumferential direction along the wall, until the ramp 710 is reached, where the flow is forced up the ramp launching or pushing the fluid towards the center of the vial 108. It is proposed, without limitations, that this creates a mixing swirl or vortex 514 in the vial. FIG. 6B shows a section front view of the vial 108 and shows a vertical feature of the ramp, namely that the ramp 710 extends from the high point 419 of the inclined bottom interior surface 416 up to about the vertical position above the outlet channel 120. The region bracketed by 716 defines a region or volume in the vial proximate to the ramp, or proximate to the inlet channel 118 and outlet channel 120. The region bracketed by 718 defines a region or volume in the vial above the ramp or the inlet channel 118 and outlet channel 120. Again without limitation, it is proposed that the ending of the ramp beyond region 716 dampens the vortex in the region 718. This dampening creates less mixing shear in region 718 than in region 716 so that particles in the solution are more likely to be maintained in area where region 718 meets region 716 which aligns with the outlet channel 120. As a person having ordinary skill would appreciate, the angle of the ramp portion, the angle of the direction vector of fluid flow 512, the density of the fluid and the particles can be varied to maintain the disbursed particles at the desired height in the vial 108 to align with the outlet channel 120 such that the particles flow out of the outlet channel 120. For example, denser or heavier particles may require a steeper ramp angle to create a faster vortex to keep the heavier particles suspended in the vial 108.

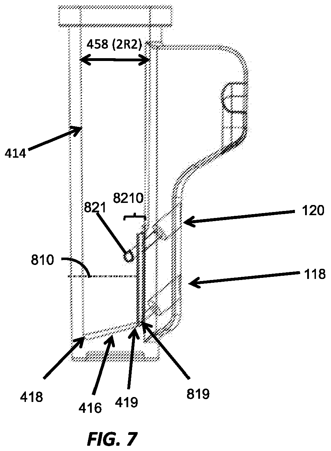

FIG. 7 shows a section view of the vial 108 illustrating the positioning of the inlet channel 118 and outlet channel 120. An opening or inlet port 819 to the inlet channel in the vial is located proximate to 419, at the top of the inclined bottom interior surface 416, allowing fluid flow to sweep along the inclined surface 416 and pick up particles that have settled along the bottom of the incline. In accordance with some embodiments, when the vial 108 is being used for administering microspheres (such as radiomicrospheres) to a patient, the level of particles, when the carrier solution is in a quiescent state (e.g., the particles are settled in bottom of the vial 108), should be below the exit port 821 to outlet channel 120. For example, the level of the microspheres can be between the low end 418 and the level of the exit port 821, and preferably up to the level indicated by dotted line 810. In accordance with some embodiments, the exit port 821 is located higher than or equal to the maximum anticipated height of particles in the vial 108.

In some embodiments, the outlet channel 120 extends into the inner volume of the vial 108 so that the opening 821 is disposed a perpendicular distance 8210 from wall 414. In some embodiments the distance 8210 is less than or equal to the radius of curvature R, which as previously defined is half of the diameter 458. For example, the distance 8210 can be in a range of 0.01R to 0.99R, 0.05R to 0.95 R, 0.05R to 0.5 R, 0.1R to 0.3R. The positioning of the opening 821 can help in avoiding air entering the channel 120 as described herein. An air bubble can be present in vial 108 when the vial is in use. Generally, this air bubble will reside at the top of the vial. However, if the vial is tipped, a situation can occur where the air bubble is proximate to the exit channel 120. Accordingly, the placement of the opening 821 can aid in avoiding any air accidently entering the channel 120 due to the vial 108 being unintentionally or intentionally moved from its upright position. In some embodiments the opening 821 is formed on an extending piece couple to channel 120. For example, the extending piece can be a tube inserted into channel 120.

In some embodiments, the inner diameter of exit port 821 to the outlet channel 120 is about equal to the diameter of inlet port 819 to the inlet channel 118. In some embodiments, these inner diameters are about 0.062 inches (about 1.57 mm). In some embodiments, the diameter of inlet port 819 (e.g., the inner diameter) is smaller than the diameter of exit port 821 (e.g., the inner diameter). In some embodiments, the diameter of the inlet port 819 is smaller than the diameter of exit port 821 in a range from 1% to 80% (e.g., from about 10% to about 75%, from about 25% to about 50%). In general, the size of the inlet port 819 can be selected to be small enough to provide the desired flow velocity (e.g., reducing the inlet port 819 size increases the flow velocity, but can result in decreased flow volume) while enabling the predefined desired amount of flow through the exit port 821. For example, in some embodiments, the inlet port 819 is 50% smaller than exit port 821 and the exit port 821 diameter can be approximately the same diameter as the tubing 110 connected to the outlet port 821 in order to avoid restricting outward flow, reducing the potential for clogging of spheres, and to minimize internal pressure buildup in the vial 108 during delivery.

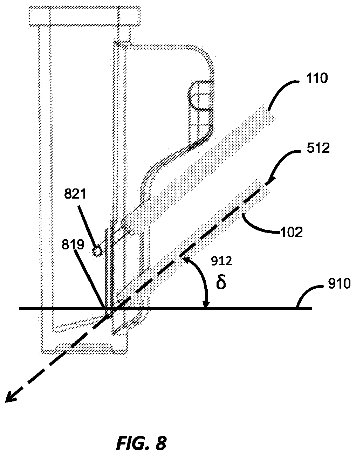

FIG. 8 illustrates the angle of the inlet channel 118 with respect to a horizontal plane 910 (parallel to plane 432 shown in FIG. 3B). The direction vector of fluid flow 512 and horizontal plane 910 define angle delta (δ) 912 which in some embodiments, is equal to or greater than angle β (β is shown in FIG. 3B). In some embodiments, δ is greater than angle β by not more than about 20° (e.g., by not more than about 10°, by not more than about 5°, or by not more than about 3°). In some embodiments, angle delta (δ) is in the range from 10° to 80° (e.g., from 20° to 70°, from 30° to 60°, from 40° to 50°,). FIG. 8 also shows tube 102 which can be coupled to input channel 118, and tube 110 which can be coupled to outlet channel 120. Without limitation, it is suggested that the downward angle δ causes fluid flow downward along the inclined base and then upward along the incline base (in a circular motion that follows the cylindrical wall 414), sweeping particles (e.g., radiomicrospheres) upward into a vortex. This design can also prevent the particles from migrating up into the inlet channel 118 and into the line 102.

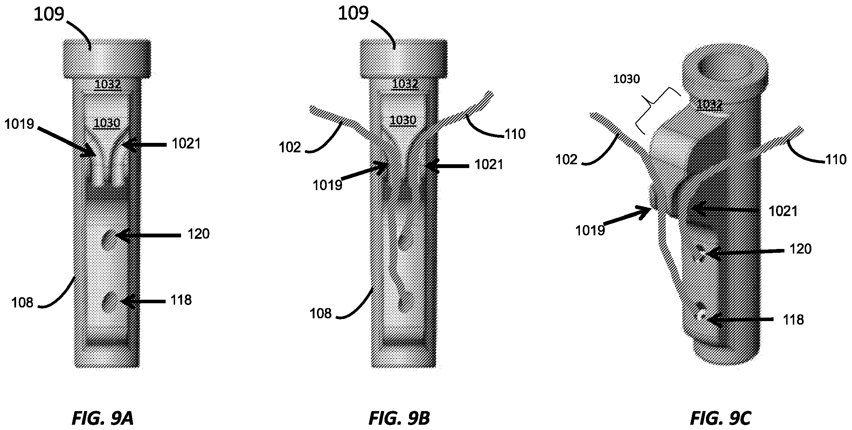

FIG 9A, 9B and 9C show several views of a vial according to some embodiments and illustrate tube management. FIG. 9A shows a front cross section view showing tube guide 1019 and tube guide 1021 that are formed in protruding feature 1030, which is a protruding feature of vial 108. FIG. 9B is a front cross section view showing line 102 positioned in tube guide 1019 and in inlet channel 118, and showing line 110 positioned in tube guide 1021 and outlet channel 120. FIG. 9C is an isometric view showing line 102 positioned in tube guide 1019 and in inlet channel 118, and showing line 110 positioned in tube guide 1021 and outlet channel 120. The tube guides 1019 and 1021 can be molded into a projecting portion on the vial 108 and can be constructed to hold the tubes in place and guide them at a controlled angle, between about 0 to 90 from a horizontal plane. This control determines the angles β (FIG. 3B) , θ (FIG 4B) and δ (FIG. 8) as previously described. Although tube guides 1019 and 1021 are shown to direct tubes in opposite directions, leftward and rightward in FIG. 9A and 9B, other embodiments can have the tube guides 1019 and 1021 directing the tubes on the same side, or independently upwards, downwards or to any side. Viewing surface 1032 between feature 1030 and cap 109 is also indicated.

FIG 9A and 9B also show a cap 109. In some embodiments this cap is crimped onto the top of the vial, positioned as shown in the figures. The cap can have a septum that can be punctured, for example, by a needle, such as a non-coring needle. This provides access to the vial for charging with microspheres, such as radiomicrospheres used for radioembolization.

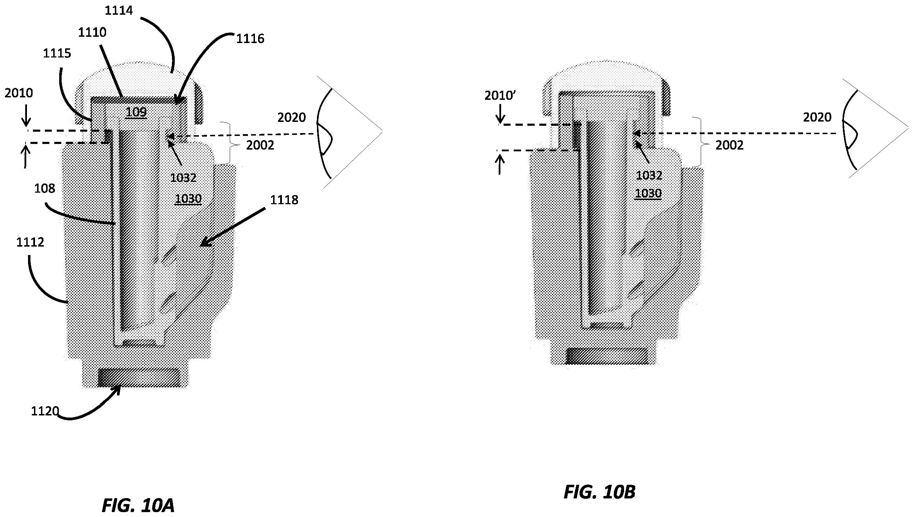

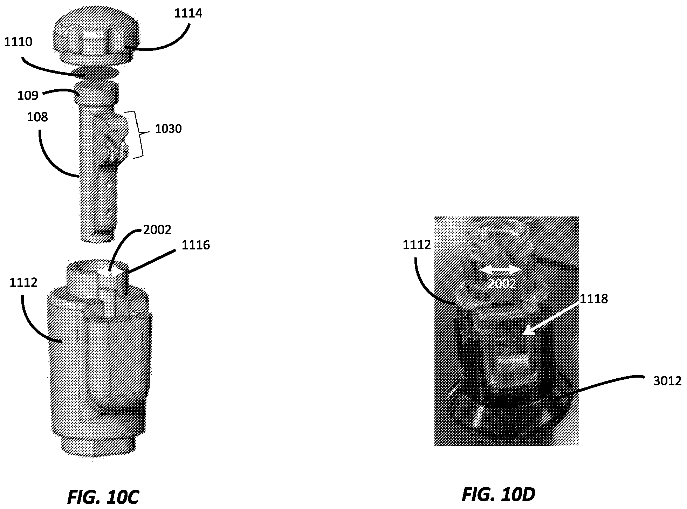

FIGS. 10A, 10B and 10C show an embodiment including a compressive sealing element 1110 in a vial holder 1112. FIG. 10A is a cross section view showing the vial 108 in the holder 1112, with a top cover 1114 compressing the sealing element 1110, against rim 1116 of the opening of the vial holder 1112. FIG. 10C is an exploded view showing the holder 1112, vial 108, sealing element 1110 and top cover 1114. In some embodiments, the top cover 1114 is a screw cap and the holder 1112 includes matching grooves or flights (not shown) close to the top 1116 of the holder. In some embodiments, the cover 1114 can be fastened to 1112 by other fastening means such as a clamp, e.g., a screw down clamp or a spring tensioned clamp. Other embodiments can include using a compression fitting, for example where wall 1117 has a taper and top cover 1114 has a matching taper. The sealing element 1110 seals against rim 1116 and against the cap 109 providing and added seal to the vial 108 such that when fluid is injected into the vial 108 vial inlet channel 118, the pressure in the vial 108 increases forcing fluid and particles (e.g. microspheres) out of the vial 108 through the outlet channel 120, and not through any opening in the cap 109, such as a needle puncture hole. Accordingly, the additional sealing element 1110 ensures that the contents in vial are not forced out and that no air enters the vial 108 where, during operation the pressure in the vial 108 changes occur (e.g., positive and negatively to atmospheric) during operation. In some embodiments, the seal 1110 can be secured (e.g., by an adhesive) to the inside surface of the cover 1114.

FIGS. 10A, 10B and 10C also illustrate a viewing port 2002 that is formed on vial holder 1112. The viewing port 2002 is configured as an elongated and wide slit or channel. Although all the material of the vial holder 1112 is transparent according to some embodiments, some light distortion and refraction can still occur. The viewing port 2002 as shown in FIG. 10B provides a clearer, less obstructed view 2020 (indicated by the dotted arrow) to the vial surface 1032 and to the contents in the vial, as compared to embodiments no featuring the viewing port 2002 as shown in FIG. 10A. In some embodiments, as shown in FIG. 10B, the vial 108 and vial holder 1112 can be elongated so that the surface 1032 is extended as indicated by dimension 2010' (as compared to dimension 2010 in FIG. 10A) and the viewing area into the vial is larger.

The viewing port can also be configured to accept the protrusion 1030 when the vial 108 is coupled to vial holder 1112, where the viewing port extends down the vial holder 1112 behind. This alignment can aid in orienting the vial 108, for example during a measurement of radioactivity, since the radiation of the vial when charged with radiomicrospheres is not uniform due to the inclined base 416, which settles the microspheres preferably in low end 418 when quiescent. A secondary holder, or cradle 3012, as shown in FIG. 10D, can also be used during this measurement, for example, to help in the orientation, stability and ensuring distance to the radioactive material in the vial 108 and the vial holder 1112 is controlled during operator manipulations and handling.

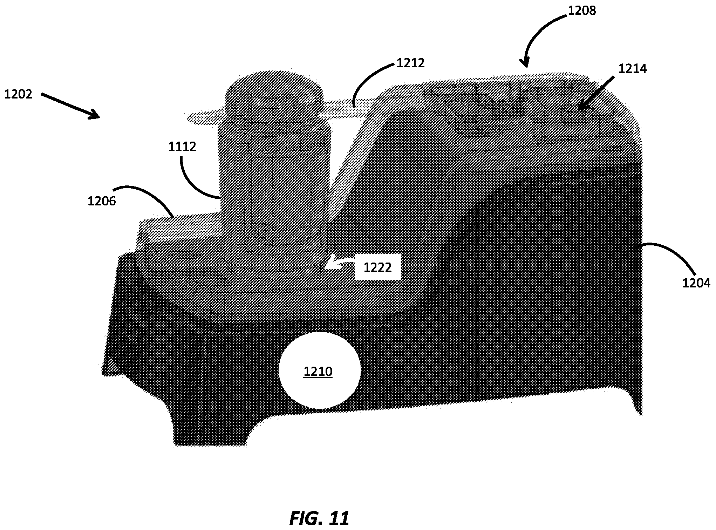

FIG. 10A, 10B, and 10C also show that holder 1112 can include a feature 1118 including a space therein (e.g., the space being between the vial 108 outer surface and an inner surface of the vial holder 1112, wherein tubing can be routed. The holder can provide radiation shielding to protect the radiologist, patients or other people during use of the vial 108, for example during a radioembolization procedure. In some embodiments, the holder is made using a radiation opaque material such as acrylic, wherein the thickness is sufficient for at least about 50% attenuation of ionizing radiation emanating from within the vial. Other materials or coatings can also be used or coated on the holder or used in construction of the holder. For example, high Z materials such as lead can be included. In some embodiments, the holder is at least about 50% transparent to visible light. As shown in FIG. 10A, the holder 1112 can include a recess 1120 which can be configured to mate with a holder 1201 as shown in FIG. 11. In accordance with some embodiments, all materials in which the microspheres are held, including the vial 108 and holder 1112, can be as transparent to visible light as possible, allowing the user to visualize the vortex and determine when the fluid's microsphere content is sufficiently diluted to end treatment. In accordance with some embodiments, the thickness of the vial 108 walls 414 can be in a range from 1/4 in. to 1/2 in. and/or the thickness of the holder 1112 walls can be in in a range from 1/4 in. to 1/2 in, where at least one of the vial 108 and the holder 1112 includes materials that are at least partially opaque to ionizing radiation.

FIG. 11 shows an isometric view of a housing 1202 for holding the vial holder 1112 and the lines of tubing according to some embodiments. The housing 1202 can include a base 1204, an opening for a light source 1210, a fluid containment ridge 1206 and a tray 1208. The base 1204 can be configured for holding the vial holder 1112 with an alignment hole 1222 in the base (or an alignment post in the base that mates with the recess 1120 in the holder 1112). In some embodiments, tray 1208 is configured for removable placement on the base and can include features 1214 for matching placement on the base, and recessed features 1306 for receiving the lines of tubing and connectors such as the three-way connection 115 and the lines of tubing 102, 106, 110, and 112 on the base 1204. In some embodiments, tray 1208 can also include an extension 1212 for coupling the tray 1208 to the vial holder 1112 and stabilizing the vial holder 1112 on the base 1204. Tray 1208 and vial holder 1112 can be surrounded by the fluid containment ridge 1206 which is configured to contain liquid that is unintentionally spilled from the vial or tubing lines. The opening 1210 is configured for accepting/insertion of a light source therein and lighting up the vial and holder through an opening 1222 in the base 1204. In some embodiments, wherein the holder 1202 and vial 108 are transparent to visible light, the light source allows visualization of the contents in the vial, such as radiomicrospheres, and monitoring how these radiomicrospheres are depleted as a radioembolization procedure progresses.

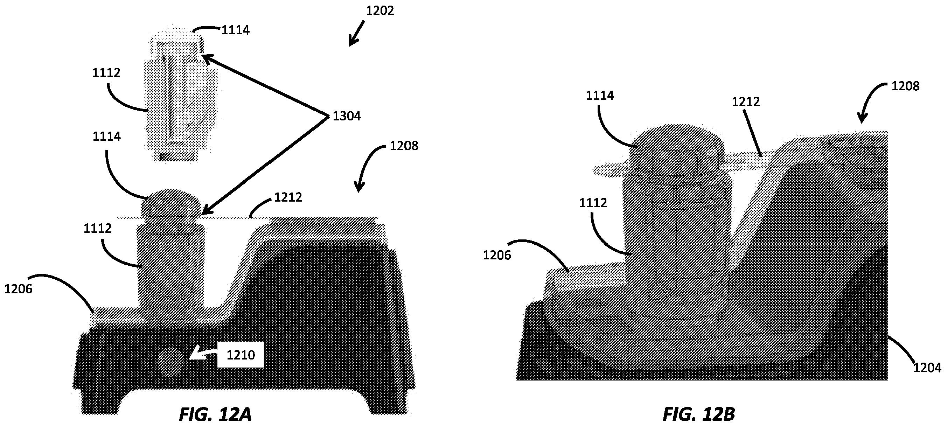

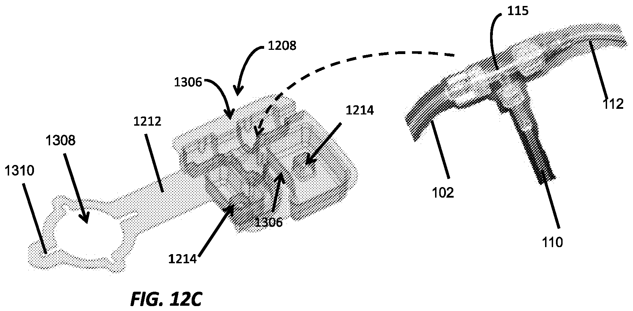

FIGS. 12A, 12B and 12C show details of the tray 1208. FIG. 12A shows a side view of the holder 1202 showing the tray 1208 and the extension 1212 of the tray which can engage with vial holder 1112. FIG. 12A also shows a section view of the vial holder for further illustration. The extension 1212 removably engages the neck region 1304 of vial holder 1112 and helps stabilize the vial holder 1112 on the base 1204. FIG. 12B is an isometric view of the vial holder 1112 on base 1204. FIG. 12C shows an isometric view of the tray 1208 as well as the three-way connection or T-connector 115. In addition to features 1214 for engaging with matching features on the base, tray 1208 includes recessed features 1306 for receiving the lines of tubing and connectors (e.g., T-connectors and associated couplers and tubing). The tray extension 1212 includes a hole 1308 for placement of neck region 1304 therethrough, and can include relief cuts 1310 allowing the tray to slip over the cap (e.g., without removing the cap).

In some embodiments, the tray 1208 is integrated as part of the base 1204 of housing 1202, such that the tray is not configured to be removable from the base 1204. In these embodiments, for example, the features such as recessed feature 1214 form a part of the base 1204. Other features such snap-fit components, cleats and fasteners for placement and securing of lines and the three-way connector 115 can also be used. Although shown as a T valve in FIG. 12C, the three-way connector 115 can be configured as a Y valve, or any other three-way connector. The recessed feature 1308 and any other snap-fit components, cleats or fasteners would be accordingly configured to couple to the three-way valve, such as a Y valve.

In addition to helping in managing the tubing and vial while in operation, in some embodiments the tray is configured to be disposed of with the vial, vial holder and associated tubing and patient interface (e.g., a microcatheter) once a procedure is completed. For example, the tray with attached tubing and vial holder can be lifted off of the base 1204 and disposed of appropriately, for example, into a radioactive waste container.

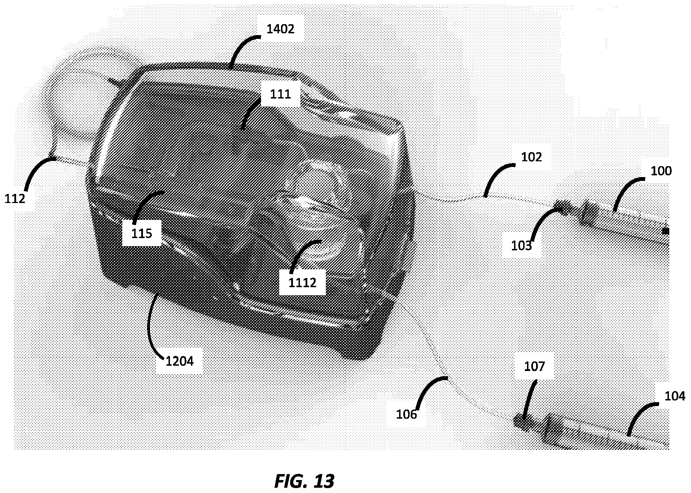

FIG. 13 shows an isometric view of the system 10 according to some embodiments. The system 10 can include the holder 1202 including a transparent cover 1402, syringe 100 connected to tubing line 102 through one-way valve 103 and syringe 104 connected to tubing line 106 and one-way valve 107. In some embodiments one-way valve 107 is positioned along line 106 so that it can be placed within cover 1402. In some embodiments one-way valve 103 is positioned along line 102 so that it can be placed within cover 1402 (e.g., attached to 3-way connector 115). The system 10 can also include a vial holder 1112 supported on the base 1204, a microcatheter line 112, a three-way connector 115, optionally configured as a T valve, and one-way valve 111.

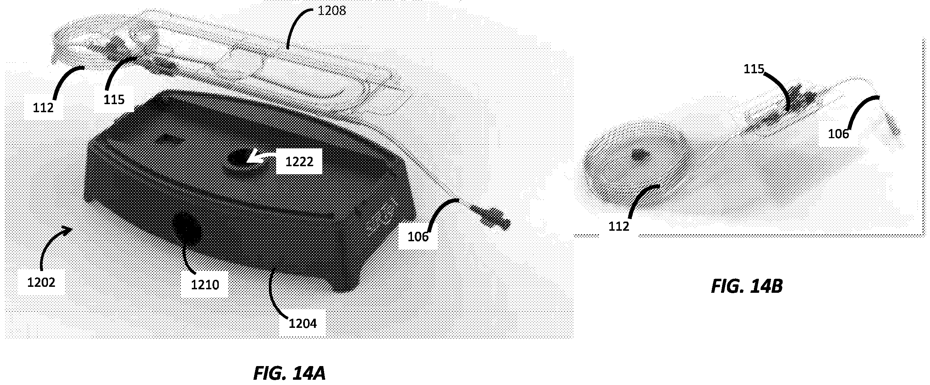

FIG. 14A shows another embodiment of a holder 1202 including a base 1204, an opening for a light source 1210, a hole in the base 1212 and a tray 1208. The tray 1208 is shown with microcatheter tube line 112, tube line 106 and T-connector 115 placed therein and hovering above the base in a depiction of assembly of the tray onto the base. FIG. 14B shows the tube 112, tube 106 and a three-way connector 115, optionally configured as a Y valve.

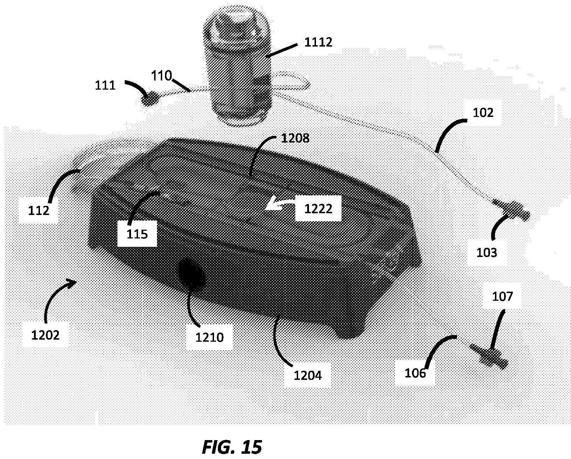

FIG. 15 is another view of the holder 1202 depicted in FIG. 14B showing a vial holder 1112 and associated tubes 110 and 102 hovering above the tray 1208 and base 1204.

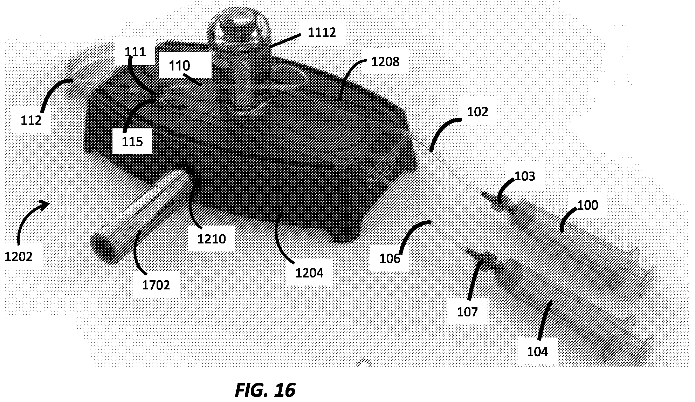

FIG. 16 is another view of holder 1202 depicted in FIG. 15, showing the placement of syringe 100 and syringe 104. The figure also shows light source 1702, proximate to aperture 1210 where it is inserted when used.

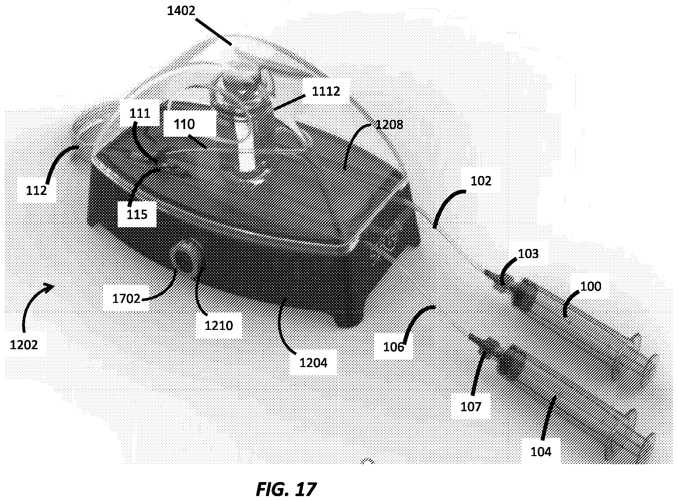

FIG. 17 is an isometric view of holder 1202 fully assembled including a transparent cover 1402 according to some embodiments. As in the embodiment depicted by FIG 13, the tray 1208 with the vial and vial holder 1112 and associated tube line 102, tube line 106, tube line 112, T-connector 115 and associated patient interface (such as a microcatheter-not shown) can be disposed of, for example as radioactive waste once a procedure is completed. The base 1202 can then be re-used with a new vial, vial holder and associated tubing, valves and connectors. In some embodiments one-way valve 107 is positioned along line 106 so that it can be placed within cover 1402. In some embodiments one-way valve 103 is positioned along line 102 so that it can be placed within cover 1402 (e.g., attached to 3-way connector 115).

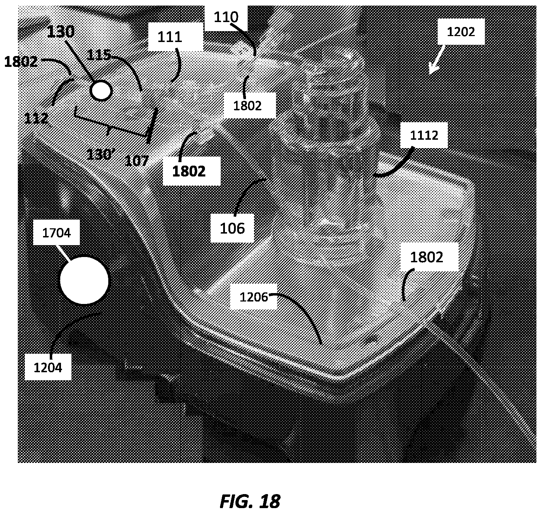

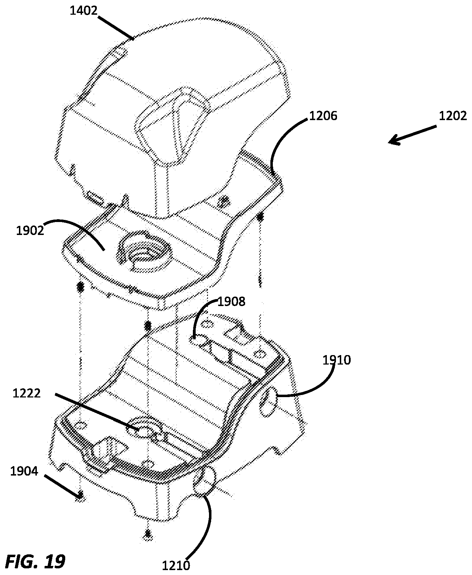

FIGS. 18 and 19 show diagrammatic isometric views of a holder 1202 according to some embodiments of the invention. The holder 1202 has features such as snap-fit components or cleats 1802 integrated with the base 1204. The snap-fit components or cleats 1802 are configured for removably holding the various tubing lines, such as 106, 110 and 112, as well as Y valve 115. In addition to snap-fit components or cleats 1802, indicia (not shown) can also be provided on the top surface of the holder 1201 aiding the operator in placement of tubes and valves. Although not shown in FIG. 18, other features such as transparent cover 1402 can be used with this embodiment. FIG. 18 also exemplifies an embodiment wherein one way valves 107 and 111 are directly attached to the Y valve 115. A bubble trap 130 can, in some embodiments, be placed in the region 130' along line 112. In some embodiments, a second light 1704 can be provided to illuminate the region 130' and/or the three-way connector 115. For example second light 1704 can be placed in base 1204 and positioned so us to illuminate upwards towards bubble trap 130.

FIG. 19 is an expanded diagrammatic view of the holder 1202 without the attached tubing showing some additional features according to some embodiments. The base 1204 can be covered with a top panel 1902 which is permanently fastened with fasteners to the base, for example, using screws 1904. The fluid containment ridge 1206 can be formed as part of the top panel 1902. In addition to an opening 1210 for a light source 1702, this embodiment shows a second opening 1910 for a light source 1704. Like opening 1210, opening 1910 can be configured to receive a light source to be place therein and to illuminate through hole or window 1908. In some embodiments, a mirror or other reflector can be provided in opening 1210 and 1910 to illuminate the fluid containing elements held by the top panel 1902. The light sources, for example, can be configured to illuminate the vial 108 in vial holder 1112 and the region 130' and/or three-way connector 115, shown in FIG. 18. For this kind of illumination, the top panel 1902 can be either transparent, or semi-transparent (e.g., at least 50% of incident light passes therethrough), or include a window or opening aligned with the openings or windows 1222 and 1908.

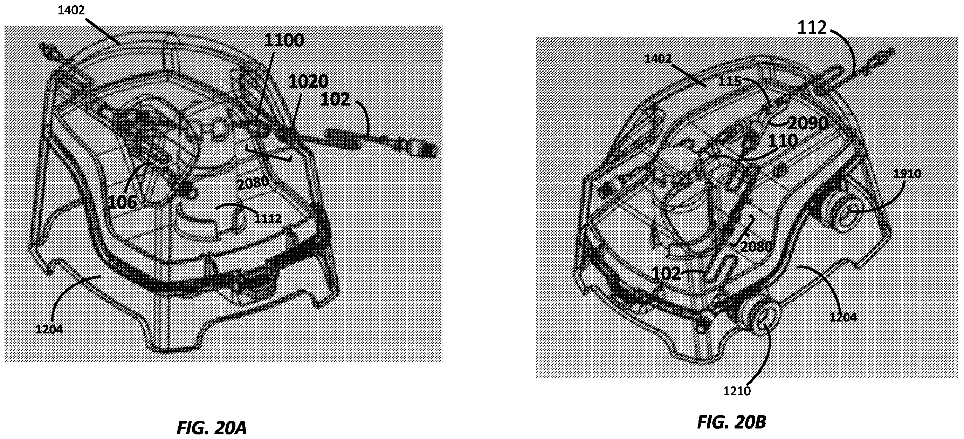

FIG. 20A and 20B show isometric views of an apparatus for delivering radiomicrospheres. Many of the features have been described herein and are labeled appropriately. In FIG. 20B the lines 102 and 110 connected to each other in at region 2080, while in FIG. 20A these are not connected to each other. The couplers on lines 102 and 110 are configured to attach to each other, for example for priming all lines through 3-way connector 115 and 112 to the microcatheter prior to microsphere delivery. These can then be disconnected, as shown in FIG. A, and then coupling element 1020 on line 102 can be connected to the inlet 118 of the vial 108, and coupling element 1100 on line 110 can be connected to outlet channel 120 of the vial 108. Also shown in FIG. 20B is indicia 2090 for placement of the 3-way connector 115.

As used herein the term "comprising" or "comprises" is used in reference to compositions, methods, and respective component(s) thereof, that are essential to the claimed invention, yet open to the inclusion of unspecified elements, whether essential or not.

As used herein the term "consisting essentially of" refers to those elements required for a given embodiment. The term permits the presence of elements that do not materially affect the basic and novel or functional characteristic(s) of that embodiment of the claimed invention.

The term "consisting of" refers to compositions, methods, and respective components thereof as described herein, which are exclusive of any element not recited in that description of the embodiment.

As used in this specification and the appended claims, the singular forms "a," "an," and "the" include plural references unless the context clearly dictates otherwise. Thus for example, references to "the method" includes one or more methods, and/or steps of the type described herein and/or which will become apparent to those persons skilled in the art upon reading this disclosure and so forth.

## Claims

1. A vial comprising;
a bottom interior surface (416) at least a portion of which has an incline angle to a horizontal plane greater than 0 degrees and a cylindrical interior wall substantially perpendicular to the horizontal plane and meeting the bottom interior surface, wherein the bottom surface and cylindrical wall form a container for containing a liquid;
an outlet channel (120) for fluid flow out of the vial through the cylindrical wall;
an inlet channel (118) for fluid flow into the vial through the cylindrical wall, the inlet channel defining a direction vector of fluid flow, wherein the direction vector is oriented at a first predefined angle with respect to the horizontal plane and oriented at a second predefined angle with respect to a line tangent to the cylindrical interior wall such that fluid flowing in through the inlet channel causes an upwardly moving fluid vortex to be created in the vial.

2. The vial according to claim 1, wherein the inlet channel is disposed at a vertical position closer to the bottom interior surface than the outlet channel.

3. The vial according to claim 1 or 2, wherein the cylindrical wall includes a ramp portion that extends inwardly from the cylindrical wall and further defines an interior surface that pushes rotational flow in the vial toward a center of the vial and wherein the ramp portion extends vertically from a position proximate to the bottom interior surface to a first predefined vertical position with respect to the outlet channel.

4. The vial according to claims 1 - 3 wherein the ramp portion is configured to produce a higher mixing shear in a solution in a first portion of the vial in a region proximate to the inlet and outlet as compared to lower mixing shear in a solution in a second portion of the vial in regions further from the inlet and outlet.

5. The vial according to any one of the above claims wherein the inlet channel comprises an inner diameter equal to or smaller than an inner diameter of the outlet channel (e.g., 1% to 80%, from about 10% to about 75%, from about 25% to about 50% smaller).

6. The vial according to any of the above claims, wherein
the bottom interior surface is approximately flat and the first predefined angle is in a range from about 10 degrees to 60 degrees (e.g., in a range from 20-25 degrees) with respect to the horizontal plane.

7. The vial according to claim 6, wherein the second predefined angle of the direction vector of fluid flow through the inlet is equal to or greater than 0° (e.g., between 0 and 20°, between 0 and 10°) with respect to the line tangent to cylindrical wall.

8. The vial according to any one of claims 1-4, wherein
the vial has a convex interior bottom surface, and
wherein a radius of curvature of the cylindrical wall is greater than a radius of curvature of the convex bottom surface.

9. A delivery device comprising:
a vial according to any one of claims 1-8,
a first syringe in fluid connection to the inlet of the vial, wherein the vial is in fluid connection to a three-way fluid connector through the outlet channel to the vial;
a second syringe in fluid connection to the three-way fluid connector;
and a patient interface in fluid connection to the three-way fluid connector; wherein,
the device is configured for delivery of:
(a) a first solution from the first syringe, through the vial, through the three-way fluid connector, and through the patient interface to a patient, and
(b) a second solution from the second syringe, through the three-way connector, and through the patient interface to a patient, and
wherein the inlet channel is disposed for delivering the first solution to the vial at the first predefined angle such that settled particles in a particle-containing solution in the vial are dispersed in the solution and exit the vial through the outlet channel.

10. The delivery device according to claim 9, further comprising a selecting-valve integrated with the three-way fluid connector for selective delivery of the first solution from the first syringe through the selecting valve to the patient or for selective delivery of the second solution from the second syringe through the selecting valve to the patient.

11. The delivery device according to claim 10, wherein the selecting-valve integrated with the three-way fluid connector is configured as a three-way stopcock.

12. The delivery device according to claim 9, further comprising a first one-way valve disposed between the first syringe and the inlet channel, and a second one-way valve disposed between the second syringe the three-way connector, wherein
the first one-way valve allows fluid flow from the first syringe to the vial, and the second one-way valve allows fluid flow from the second syringe to the three-way fluid-connector.

13. The delivery device according to claim 12, further comprising a third one-way valve disposed between the vial outlet and the three-way fluid connector, wherein the third one-way valve allows fluid and particle flow from the vial to the patient interface when a fluid pressure on an inlet of the third one-way valve is at least about 0.2 psi above a pressure on an outlet of the third one-way valve.

14. The delivery device according to any one of claims 9-13, further including a vial holder configured as a container having a top opening and a removable top cover, and wherein the container is configured to receive the vial therein through the top opening, and the removeable top cover is configured for compressing an elastomeric seal against an opening at the top of the vial when the top cover is attached to the container, thereby providing a fluid seal at the top opening of the vial, and
the inlet channel is provided with fluid connection to the first syringe using a first tube, the outlet channel is provided with fluid connection to the three-way valve using a second tube, and the vial holder includes an opening for allowing connection of the first tube to the inlet channel, and an opening for allowing connection of the second tube to the outlet channel.

15. The delivery device according to claim 14 further comprising a light disposed to illuminate the interior of the vial and wherein the vial and vial container are at least 80% transparent to the light.

16. The delivery device according to claim 14 or 15, wherein the container and vial are at least partially opaque to ionizing radiation (e.g., such that at least 50% of the radiation emanating from the interior of the vial is attenuated by the vial and container).

17. The delivery device according to any one of claims 14-16, further comprising a holder for the vial and vial holder, wherein the holder includes,
a moat channel surrounding the vial and configured for containing at least the volume of the interior of the vial,
a transparent lid for enclosing the vial and three-way fluid connector, and
a removable tray configured for attachment to the holder and configured for holding the three-way fluid connector and removably attaching to the vial holder.

18. The delivery device according to claim 17, wherein the fluid connection from the second syringe to the three-way fluid connector is provide by removable attachment to a third tube, the fluid connection from the three-way fluid connector to the patient interface is provide by removable attachment to a fourth tube, wherein at least a portion of the first, third and fourth tubes are placed in the holder when the device is in operation.

19. The delivery device according to any one of the above claims wherein the patient interface is a microcatheter.

## Patentansprüche

1. Ampulle, umfassend;
eine untere Innenfläche (416), von der zumindest ein Teil einen Neigungswinkel zu einer waagerechten Ebene von mehr als 0 Grad aufweist, und eine zylindrische Innenwand, die im Wesentlichen rechtwinklig zu der waagerechten Ebene ist und auf die untere Innenfläche trifft, wobei die untere Fläche und die zylindrische Wand einen Behälter zum Aufnehmen einer Flüssigkeit bilden;
einen Auslasskanal (120) für die Fluidströmung aus der Ampulle durch die zylindrische Wand;
einen Einlasskanal (118) für die Fluidströmung in die Ampulle durch die zylindrische Wand, wobei der Einlasskanal einen Richtungsvektor der Fluidströmung definiert, wobei der Richtungsvektor derartig in einem ersten vordefinierten Winkel in Bezug auf die waagerechte Ebene ausgerichtet ist und in einem zweiten vordefinierten Winkel in Bezug auf eine die zylindrische Innenwand berührende Gerade ausgerichtet ist, sodass Fluid, das durch den Einlasskanal einströmt, bewirkt, dass ein sich nach oben bewegender Fluidwirbel in der Ampulle erzeugt wird.

2. Ampulle nach Anspruch 1, wobei der Einlasskanal an einer senkrechten Stellung angeordnet ist, die sich näher an der unteren Innenfläche befindet als der Auslasskanal.

3. Ampulle nach Anspruch 1 oder 2, wobei die zylindrische Wand einen Rampenabschnitt beinhaltet, der sich von der zylindrischen Wand nach innen erstreckt und ferner eine Innenfläche definiert, die eine Rotationsströmung in der Ampulle zu einer Mitte der Ampulle drückt, und wobei sich der Rampenabschnitt senkrecht von einer Stellung nahe der unteren Innenfläche zu einer ersten vordefinierten senkrechten Stellung in Bezug auf den Auslasskanal erstreckt.

4. Ampulle nach den Ansprüchen 1-3, wobei der Rampenabschnitt dazu konfiguriert ist, in einer Lösung in einem ersten Abschnitt der Ampulle in einem Bereich nahe dem Einlass und Auslass eine höhere Mischscherung im Vergleich zu einer niedrigeren Mischscherung in einer Lösung in einem zweiten Abschnitt der Ampulle in Bereichen, die weiter vom Einlass und Auslass entfernt sind, zu produzieren.

5. Ampulle nach einem der vorstehenden Ansprüche, wobei der Einlasskanal einen Innendurchmesser umfasst, der gleich einem oder kleiner als ein Innendurchmesser des Auslasskanals ist (z. B. 1 % bis 80 %, von etwa 10 % bis etwa 75 %, von etwa 25 % bis etwa 50 % kleiner).

6. Ampulle nach einem der vorstehenden Ansprüche, wobei die untere Innenfläche annähernd eben ist und der erste vordefinierte Winkel in einem Bereich von etwa 10 Grad bis 60 Grad (z. B. in einem Bereich von 20-25 Grad) in Bezug auf die waagerechte Ebene liegt.

7. Ampulle nach Anspruch 6, wobei der zweite vordefinierte Winkel des Richtungsvektors der Fluidströmung durch den Einlass gleich oder größer als 0° (z. B. zwischen 0 und 20°, zwischen 0 und 10°) in Bezug auf ist die die zylindrische Wand berührende Gerade ist.

8. Ampulle nach einem der Ansprüche 1-4, wobei
die Ampulle eine konvexe untere Innenfläche aufweist, und wobei ein Krümmungsradius der zylindrischen Wand größer als ein Krümmungsradius der konvexen unteren Fläche ist.

9. Abgabevorrichtung, umfassend:
eine Ampulle nach einem der Ansprüche 1-8,
eine erste Spritze in Fluidverbindung mit dem Einlass der Ampulle, wobei die Ampulle mit einem Dreiwegefluidanschluss in Fluidverbindung durch den Auslasskanal zu der Ampulle steht;
eine zweite Spritze in Fluidverbindung mit dem Dreiwegefluidanschluss;
und eine Patientenschnittstelle in Fluidverbindung mit dem Dreiwegefluidanschluss; wobei
die Vorrichtung für die Abgabe von Folgendem konfiguriert ist:
(a) eine erste Lösung aus der ersten Spritze, durch die Ampulle, durch den Dreiwegefluidanschluss und durch die Patientenschnittstelle zu einem Patienten, und
(b) eine zweite Lösung aus der zweiten Spritze, durch den Dreiwegeanschluss und durch die Patientenschnittstelle zu einem Patienten, und
wobei der Einlasskanal derartig zum Abgeben der ersten Lösung an die Ampulle in dem ersten vordefinierten Winkel angeordnet ist, dass abgesetzte Partikel in einer Partikel enthaltenden Lösung in der Ampulle in der Lösung dispergiert werden und die Ampulle durch den Auslasskanal verlassen.

10. Abgabevorrichtung nach Anspruch 9, ferner umfassend ein in den Dreiwegefluidanschluss integriertes Auswahlventil zur wahlweisen Abgabe der ersten Lösung aus der ersten Spritze durch das Auswahlventil an den Patienten oder zur wahlweisen Abgabe der zweiten Lösung aus der zweiten Spritze durch das Auswahlventil an den Patienten.

11. Abgabevorrichtung nach Anspruch 10, wobei das in den Dreiwegefluidanschluss integrierte Auswahlventil als ein Dreiwege-Absperrhahn konfiguriert ist.

12. Abgabevorrichtung nach Anspruch 9, ferner umfassend ein erstes Einwegventil, das zwischen der ersten Spritze und dem Einlasskanal angeordnet ist, und ein zweites Einwegventil, das zwischen der zweiten Spritze und dem Dreiwegeanschluss angeordnet ist, wobei
das erste Einwegventil eine Fluidströmung von der ersten Spritze zu der Ampulle ermöglicht, und das zweite Einwegventil eine Fluidströmung von der zweiten Spritze zu dem Dreiwegefluidanschluss ermöglicht.

13. Abgabevorrichtung nach Anspruch 12, ferner umfassend ein drittes Einwegventil, das zwischen dem Ampullenauslass und dem Dreiwegefluidanschluss angeordnet ist, wobei das dritte Einwegventil eine Fluid- und Partikelströmung von der Ampulle zu der Patientenschnittstelle ermöglicht, wenn ein Fluiddruck an einem Einlass des dritten Einwegventils mindestens etwa 0,2 psi über einem Druck an einem Auslass des dritten Einwegventils liegt.

14. Abgabevorrichtung nach einem der Ansprüche 9-13, ferner beinhaltend einen Ampullenhalter, der als ein Behälter mit einer oberen Öffnung und einer abnehmbaren oberen Abdeckung konfiguriert ist, und wobei der Behälter dazu konfiguriert ist, die Ampulle darin durch die obere Öffnung aufzunehmen, und die abnehmbare obere Abdeckung zum Pressen einer Elastomerdichtung gegen eine Öffnung an der Oberseite der Ampulle konfiguriert ist, wenn die obere Abdeckung an dem Behälter angebracht ist, wodurch eine Fluiddichtung an der oberen Öffnung der Ampulle bereitgestellt wird, und
der Einlasskanal unter Verwendung eines ersten Schlauchs mit einer Fluidverbindung zur ersten Spritze bereitgestellt ist, der Auslasskanal unter Verwendung eines zweiten Schlauchs mit einer Fluidverbindung zum Dreiwegeventil bereitgestellt ist, und der Ampullenhalter eine Öffnung zum Ermöglichen einer Verbindung des ersten Schlauchs zu dem Einlasskanal und eine Öffnung zum Ermöglichen einer Verbindung des zweiten Schlauchs mit dem Auslasskanal beinhaltet.

15. Abgabevorrichtung nach Anspruch 14, ferner umfassend eine Leuchte, die dazu angeordnet ist, das Innere der Ampulle zu beleuchten, und wobei die Ampulle und der Ampullenbehälter für die Leuchte zu mindestens 80 % lichtdurchlässig sind.

16. Abgabevorrichtung nach Anspruch 14 oder 15, wobei der Behälter und die Ampulle mindestens teilweise undurchlässig für ionisierende Strahlung sind (z. B. derartig, dass mindestens 50 % der Strahlung, die von dem Inneren der Ampulle ausgeht, durch die Ampulle und den Behälter abgeschwächt wird).

17. Abgabevorrichtung nach einem der Ansprüche 14-16, ferner umfassend einen Halter für die Ampulle und den Ampullenhalter, wobei der Halter Folgendes beinhaltet:
einen Grabenkanal, der die Ampulle umgibt und dazu konfiguriert ist, mindestens das Volumen des Inneren der Ampulle zu enthalten,
einen lichtdurchlässigen Deckel zum Umschließen der Ampulle und des Dreiwegefluidanschlusses, und
ein abnehmbares Tablett, das zur Anbringung an dem Halter und zum Halten des Dreiwegefluidanschlusses und zur lösbaren Anbringung an dem Ampullenhalter konfiguriert ist.

18. Abgabevorrichtung nach Anspruch 17, wobei die Fluidverbindung von der zweiten Spritze zum Dreiwegefluidanschluss durch abnehmbare Anbringung an einem dritten Schlauch bereitgestellt ist, wobei die Fluidverbindung vom Dreiwegefluidanschluss zur Patientenschnittstelle durch abnehmbare Anbringung an einen vierten Schlauch bereitgestellt ist, wobei mindestens ein Abschnitt des ersten, dritten und vierten Schlauchs in dem Halter angeordnet sind, wenn die Vorrichtung in Betrieb ist.

19. Abgabevorrichtung nach einem der vorstehenden Ansprüche, wobei die Patientenschnittstelle ein Mikrokatheter ist.

## Revendications

1. Flacon comprenant ;
une surface intérieure inférieure (416) dont au moins une partie a un angle d'inclinaison par rapport à un plan horizontal supérieur à 0 degré et une paroi intérieure cylindrique sensiblement perpendiculaire au plan horizontal et rencontrant la surface intérieure inférieure, dans lequel la surface inférieure et la paroi cylindrique forment un récipient pour contenir un liquide ;
un canal de sortie (120) pour l'écoulement de fluide hors du flacon à travers la paroi cylindrique ;
un canal d'entrée (118) pour l'écoulement de fluide dans le flacon à travers la paroi cylindrique, le canal d'entrée définissant un vecteur de direction d'écoulement de fluide, dans lequel le vecteur de direction est orienté selon un premier angle prédéfini par rapport au plan horizontal et orienté selon un second angle prédéfini par rapport à une ligne tangente à la paroi intérieure cylindrique de sorte que le fluide s'écoulant à travers le canal d'entrée provoque la création d'un vortex de fluide se déplaçant vers le haut dans le flacon.

2. Flacon selon la revendication 1, dans lequel le canal d'entrée est disposé à une position verticale plus proche de la surface intérieure inférieure que le canal de sortie.

3. Flacon selon la revendication 1 ou 2, dans lequel la paroi cylindrique comporte une partie inclinée qui s'étend vers l'intérieur à partir de la paroi cylindrique et définit en outre une surface intérieure qui pousse l'écoulement rotatif dans le flacon vers un centre du flacon et dans lequel la partie inclinée s'étend verticalement depuis une position proche de la surface intérieure inférieure jusqu'à une première position verticale prédéfinie par rapport au canal de sortie.

4. Flacon selon les revendications 1 à 3, dans lequel la partie inclinée est configurée pour produire un cisaillement de mélange plus élevé dans une solution dans une première partie du flacon dans une région proche de l'entrée et de la sortie par rapport à un cisaillement de mélange plus faible dans une solution dans une seconde partie du flacon dans des régions plus éloignées de l'entrée et de la sortie.

5. Flacon selon l'une quelconque des revendications précédentes, dans lequel le canal d'entrée comprend un diamètre interne inférieur ou égal à un diamètre interne du canal de sortie (par exemple, 1 % à 80 %, d'environ 10 % à environ 75 %, d'environ 25 % à environ 50 % plus petit).

6. Flacon selon l'une quelconque des revendications précédentes, dans lequel
la surface intérieure inférieure est approximativement plate et le premier angle prédéfini est dans une plage d'environ 10 degrés à 60 degrés (par exemple, dans une plage de 20 à 25 degrés) par rapport au plan horizontal.

7. Flacon selon la revendication 6, dans lequel le second angle prédéfini du vecteur de direction d'écoulement de fluide à travers l'entrée est supérieur ou égal à 0° (par exemple, entre 0 et 20°, entre 0 et 10°) par rapport à la droite tangente à la paroi cylindrique.

8. Flacon selon l'une quelconque des revendications 1 à 4, dans lequel
le flacon a une surface inférieure intérieure convexe, et
dans lequel un rayon de courbure de la paroi cylindrique est supérieur à un rayon de courbure de la surface inférieure convexe.

9. Dispositif d'administration comprenant :
un flacon selon l'une quelconque des revendications 1 à 8,
une première seringue en communication fluidique avec l'entrée du flacon, dans lequel le flacon est en communication fluidique avec un connecteur de fluide à trois voies à travers le canal de sortie vers le flacon ;
une seconde seringue en communication fluidique avec le connecteur de fluide à trois voies ;
et une interface patient en communication fluidique avec le connecteur de fluide à trois voies ; dans lequel,
l'appareil est configuré pour administrer :
(a) une première solution depuis la première seringue, à travers le flacon, à travers le connecteur de fluide à trois voies, et à travers l'interface patient à un patient, et
(b) une seconde solution depuis la seconde seringue, à travers le connecteur à trois voies, et à travers l'interface patient à un patient, et
dans lequel le canal d'entrée est disposé pour administrer la première solution au flacon selon le premier angle prédéfini de sorte que les particules déposées dans une solution contenant des particules dans le flacon sont dispersées dans la solution et sortent du flacon par le canal de sortie.

10. Dispositif d'administration selon la revendication 9, comprenant en outre une vanne de sélection intégrée au connecteur de fluide à trois voies pour l'administration sélective de la première solution depuis la première seringue à travers la vanne de sélection au patient ou pour l'administration sélective de la seconde solution depuis la seconde seringue à travers la vanne de sélection au patient.

11. Dispositif d'administration selon la revendication 10, dans lequel la vanne de sélection intégrée au connecteur de fluide à trois voies est configurée comme un robinet d'arrêt à trois voies.

12. Dispositif d'administration selon la revendication 9, comprenant en outre une première vanne unidirectionnelle disposée entre la première seringue et le canal d'entrée, et une deuxième vanne unidirectionnelle disposée entre la seconde seringue et le connecteur à trois voies, dans lequel la première vanne unidirectionnelle permet l'écoulement de fluide de la première seringue au flacon, et la deuxième vanne unidirectionnelle permet l'écoulement de fluide de la seconde seringue au connecteur de fluide à trois voies.

13. Dispositif d'administration selon la revendication 12, comprenant en outre une troisième vanne unidirectionnelle disposée entre la sortie du flacon et le connecteur de fluide à trois voies, dans lequel la troisième vanne unidirectionnelle permet l'écoulement de fluide et de particules du flacon à l'interface patient lorsqu'une pression de fluide sur une entrée de la troisième vanne unidirectionnelle est d'au moins environ 0,2 psi au-dessus d'une pression sur une sortie de la troisième vanne unidirectionnelle.

14. Dispositif d'administration selon l'une quelconque des revendications 9 à 13, comprenant en outre un support de flacon configuré comme un récipient ayant une ouverture supérieure et un couvercle supérieur amovible, et dans lequel le récipient est configuré pour recevoir le flacon à travers l'ouverture supérieure, et le couvercle supérieur amovible est configuré pour comprimer un joint élastomère contre une ouverture sur la partie supérieure du flacon lorsque le couvercle supérieur est fixé au récipient, fournissant ainsi un joint d'étanchéité aux fluides au niveau de l'ouverture supérieure du flacon, et
le canal d'entrée est pourvu d'une communication fluidique avec la première seringue à l'aide d'un premier tube, le canal de sortie est pourvu d'une communication fluidique avec la vanne à trois voies à l'aide d'un deuxième tube, et le support de flacon comporte une ouverture pour permettre le raccordement du premier tube au canal d'entrée, et une ouverture pour permettre le raccordement du deuxième tube au canal de sortie.

15. Dispositif d'administration selon la revendication 14, comprenant en outre une lumière disposée pour éclairer l'intérieur du flacon et dans lequel le flacon et le récipient du flacon sont transparents à au moins 80 % à la lumière.

16. Dispositif d'administration selon la revendication 14 ou 15, dans lequel le récipient et le flacon sont au moins partiellement opaques au rayonnement ionisant (par exemple, de sorte qu'au moins 50 % du rayonnement émanant de l'intérieur du flacon est atténué par le flacon et le récipient).

17. Dispositif d'administration selon l'une quelconque des revendications 14 à 16, comprenant en outre un support pour le flacon et le support de flacon, dans lequel le support comporte, un canal de cavité entourant le flacon et configuré pour contenir au moins le volume de l'intérieur du flacon,
un couvercle transparent pour enfermer le flacon et le connecteur de fluide à trois voies, et
un plateau amovible configuré pour être fixé au support et configuré pour supporter le connecteur de fluide à trois voies et se fixer de manière amovible au support de flacon.

18. Dispositif d'administration selon la revendication 17, dans lequel la communication fluidique de la seconde seringue au connecteur de fluide à trois voies est prévue par une fixation amovible à un troisième tube, la communication fluidique du connecteur de fluide à trois voies à l'interface patient est prévue par fixation amovible à un quatrième tube, dans lequel au moins une partie des premier, troisième et quatrième tubes est placée dans le support lorsque le dispositif est en fonctionnement.

19. Dispositif d'administration selon l'une quelconque des revendications précédentes dans lequel l'interface patient est un micro-cathéter.
